# EUROPEAN PATENT APPLICATION

(11) **EP 2 722 062 A1**
(43) Date of publication of application: **23.04.2014**
(21) Application number: 12800524.6
(22) Date of filing: 15.06.2012
(51) Int. Cl.: A61L 31/04, A61L 31/06, A61L 31/18, A61K 33/14, A61K 33/18, C08K 3/10, C08K 3/16

(54) **LIQUID EMBOLIC MATERIALS BASED ON COLLAGENS AND PREPARATION METHOD THEREOF**

(30) Priority: 17.06.2011 CN 201110172325
(71) Applicant: Shanghai MicroPort Medical (Group) Co., Ltd., Pudong New Area Shanghai Shanghai 201203 (CN)
(72) Inventor: LENG, Bing, Shanghai 201203 (CN); KANG, Yahong, Shanghai 201203 (CN); JIN, Qiaorong, Shanghai 201203 (CN); XIE, Zhiyong, Shanghai 201203 (CN); LUO, Qiyi, Shanghai 201203 (CN)
(74) Representative: Dantz, Jan Henning
(86) International application number: PCT/CN2012/076981
(87) International publication number: WO 2012/171478

(57) **Abstract**

The present invention relates to the field of medical devices. Specifically, the present invention relates to a novel collagen-based liquid embolic material and its preparation method. The alkyl cyanoacrylate and the collagen contained in the embolic material self-assemble into a multiple-junction polymer during embolization, to thereby produce an embolic material system. In one aspect, the alkyl cyanoacrylate, as a typical liquid embolic material, has a good embolic effect; and, in another aspect, the collagen can absorb water and expand rapidly, and can induce the increase of endothelial growth factor upon embolization, which provides an embolic body with a low density and a good flexibility, as well as a permanent embolization with no recurrence.

## Description

### Technical Field

The present invention relates to the field of medical devices. More specifically, the present invention relates to a novel collagen-based liquid embolic material and its preparation method.

### Background art

Due to the effect of various external and internal factors, such as mechanical damage, vascular sclerosis, high blood pressure, vascular smooth muscle cell proliferation, bacterial or viral infections, induction of venous valve diseases, and impact of blood flow, aneurysms or other abnormalities are easy to be formed in the arteriovenous vessels. An aneurysm with a rounding convex wall is often formed intracranially, and such an intracranial aneurysm caused by the cystic dilatation of intracranial vessel wall, mostly occurs in the vicinity of Wills ring. The greatest risk associated with aneurysms is their potency to hemorrhage per rhexin, so called a "time bomb", with a mortality rate of about 1/3 for each hemorrhage per rhexin, as well as an extremely low survivability after three consecutive hemorrhages. Thus, it is extremely important to timely and properly treat the intracranial aneurysms. An arteriovenous malformation (AVM) is a congenital disorder caused by the obstructed cerebrovascular development during embryonic three to four weeks, resulting in forming a direct connection of arteries and veins. AVM is characterized by lack of capillaries between arteries and veins, which are replaced by a tangle of abnormal vessels of various diameters and wall thicknesses. These abnormal vessels lack elastic and muscular layers and are prone to hemorrhage per rhexin, with the result that AVM is one of the most disabling diseases among young people. Due to its complexity and the highest morbidity and disability rate, AVM has been a major problem in neurosurgery for years.

As the routine treatment of the intracranial aneurysm in cerebrum, existing aneurysms are usually sealed and blocked by surgery, including snipping and ligating the parent artery of aneurysm, and clamping the neck of aneurysm by a clip for aneurysm, so as to block the impact of blood flow on the aneurysm; and traditionally, the primary method for treating AVM is the craniotomy to resect the malformation. However, this method takes relatively long time and may cause damage to the brain tissue; and for some reasons, some of the patients with intracranial aneurysm or vascular malformation are not suitable for the craniotomy or unwilling to accept the craniotomy. In recent years, with the development of vascular imaging, most surgeries are being replaced by interventional therapy using interventional surgery to transport a variety of embolic materials into the cavity of aneurysm in cerebrum so as to seal, block and embolize the aneurysm. Endovascular embolic therapy has become an important treatment for the arteriovenous malformation (AVM) in cerebrum. Particularly, giant, deeply-located or complex arteriovenous malformations in cerebrum, or those with high blood flow or in functional areas are the ideal indications for endovascular therapy.

In the prior art, a variety of embolic materials have been disclosed, which can be generally divided into two categories of solid embolic material and liquid embolic material. A solid embolic material can be classified as a permanent solid embolic material or an absorbable solid embolic material, according to its degradability; and a liquid embolic material is generally classified as an adhesive liquid embolic material or a non-adhesive liquid embolic material. Since the liquid embolic material may be injected directly into the aneurysm cavity, it can adapt to various aneurysm cavities of different shapes and sizes, without leaving any gaps between the aneurysm wall and the embolic material, so as to achieve a permanent occlusion. Meanwhile, the liquid embolic material has several advantages such as easy to operate, low intraoperative risk, etc, and can be injected directly into vascular lesions through a micro-catheter, so the liquid embolic material is a relatively ideal embolic material, often used in the field of endovascular treatment for intracranial aneurysms, cerebral AVM, dural arteriovenous fistula (DAVF), spinal vascular malformations, and the like.

An ideal liquid embolic material should meet the following criteria: 1) the liquid material should have a moderate viscosity, the easiness of passing through an elongated micro-catheter, a low resistance for injection, the capability of being safely placed into vascular lesions, and the capability of meeting the purpose of embolizing vascular lesions; 2) the liquid material is easy to operate and control, and can be developed well; 3) the liquid material can be injected multiple times through a single catheter before the catheter is withdrawn when a satisfactory embolization is achieved, and the liquid material does not give sticky catheter; 4) the liquid material can be well diffused throughout the vascular malformation, capable of diffusing from the arterial end of the lesion to the entire lesion, but not reaching the venous end of the lesion; 5) the embolic effect is permanent, without vascular recanalization phenomenon; 6) the embolic material should have a long-term stability, that is to say, it has a stable embolic effect in the in-vivo environment and during the desired period, and does not cause harmful chemical reactions or physical changes; 7) the embolic material is biocompatible, non-toxic and not teratogenic or carcinogenic, with no biological activity; and 8) after being treated by embolization, the tangle of vessels of the vascular lesion become soft, and easy to be stretched and excised by surgery.

Presently, the adhesive liquid embolic material used in clinical applications is primarily n-butyl cyanoacrylate. The n-butyl cyanoacrylate has a strong adhesiveness and is prone to cause "sticky catheter" problem, that is to say, the micro-catheter sticks to the vessel wall, resulting in foreign materials being left in the body, which have certain risks and may produce complications due to "sticky catheter" during embolization. The non-adhesive liquid embolic material is primarily onyx glue or the like, wherein the organic solvent used, i.e., DMSO has potential vascular toxicity, and thus becomes the major obstacle to their wide application. Meanwhile, the innate properties of onyx make it impossible for onyx to be closely bonded with the vascular wall at lesions. Clinical data shows that the half-year recurrence rate of aneurysms, especially large or giant aneurysms after being embolized with onyx is nearly 78%. As can be seen, the existing liquid embolic materials do not completely meet the above criterions, and thus there is a need for a novel embolic material, which not only does not cause cellular toxicity or sticky catheter problem, but also enables the vascular disease after embolization to be permanently occluded with no recurrence.

CN1413743 discloses a novel vascular embolic material comprising an embolic material and an analgesic which is blended into the embolic material or attached onto the embolic material to form a granular structure or block structure or linear structure or spring-like structure, for effectively preventing ischemic pain in the target lesion of the patient after vascular embolization by the slow release of the analgesic.

CN1449741 discloses an easy-to-operate and quality-stable embolic material by providing an embolic material containing an ester derivative of polysaccharide with a weight average molecular weight of 10,000 - 500,000 daltons and an average substitution degree of acyl of more than 2; an embolic formulation suitable for arteriovenous aneurysm, comprising the embolic material and a physiologically acceptable and suitable water-soluble organic acid; and the process for producing the embolic formulation, comprising the step of dissolving the embolic material in the physiologically acceptable and suitable water-soluble organic solvent.

CN86107191 discloses a vascular embolic formulation (TH glue) prepared from n-octyl alpha-cyanoacrylate, tantalum powder and iodized oil (myodil) at the ratio of volume percentages of 80-85: 1-5: 10-15, preferably 83.75: 2.5: 13.75, wherein the single component has a good stability with convenience of use; and the preparation method thereof. The formulation is a permanent vascular embolic material which aggregates rapidly *in vivo* accompanying with low exotherm, resulting in a soft aggregate which cannot be absorbed by blood or tissue fluid, or degradated, so as to lose its efficacy, and can be developed *in vivo* under the irradiation of X-ray. The embolic material may be used for injection by glass injectors

In addition, the prior art also discloses an experimental study on collagen coil embolization of intracranial aneurysms in rabbits. The conclusion is that obvious cellular responses, progressive thrombosis and dense connective tissue matrix deposition may occur after collagen coil embolization; while in the platinum coil embolization group of animal models, merely loose connective tissue matrix deposition is observed, with rare progressive thrombosis (see: Chinese Journal of Minimally Invasive Neurosurgery (CMINSJ), 2009, 14(11)).

Collagen is known as a biopolymer (extracellular protein), with the most common structural feature of triple helical structure. It is consisted of three α-chain polypeptides, each possessing the conformation of a left-handed helix. These three left-handed helices are twisted together into a right-handed helical structure, i.e., super helical structure, a triple helical structure in a fibrillar form unique to the collagen, enabling a very stable molecular structure, a low immunogenicity, a good biocompatibility and the like. Due to the presence of numerous hydrophilic groups on the molecular chains, collagen has a strong ability of binding with water, and is able to expand in acidic and alkaline media, resulting in forming a web consisted of elongated fibers.

However, the use of the collagen combined with cyanoacrylates and the beneficial effects thereof have never been reported in the prior art.

### Summary of the Invention

To solve the above technical problem, the present invention provides a novel collagen-based liquid embolic material, wherein the alkyl cyanoacrylate and the collagen contained therein self-assemble into a multiple-junction polymer during embolization, to thereby produce an embolic material system.

The embolic material of the present invention may be further mixed with a vascular developer material (contrast agent), to be suitable for visibly observing the embolization of arteriovenous aneurysm by fluorescent X-ray radiography during and after surgery, wherein the vascular developer material may be any one of those conventionally used in angiography, without any particular limitation. Examples of the contrast agent are: iodide, including 6-triiodo formic acid, sodium 6-triiodobenzoate, iothalamic acid, metrizoic acid, iodamide, ioxaglic acid, iopanoic acid, iohexol, and iotrolan; bismuth-containing compound, such as bismuth trioxide; metal powder, such as tantalum powder, gold powder, tungsten powder, or the like; and any mixture thereof.

The present invention further provides a method for preparing the foresaid novel liquid embolic material, comprising: granulating the collagen, and adding and uniformly dispersing the granulated collagen into alkyl cyanoacrylate monomers, to thereby form the novel liquid embolic material. In a preferred embodiment, the preparation method of the present invention further comprises adding a vascular developer material, for example, adding metal powder at 37-50°C. Through a micro-catheter, the mixed system is slowly introduced into a target area. With the concentration of monomers increasing, the rapid expansion of collagen and the anionic polymerization of cyanoacrylate are gradually achieved in the plasma under slightly alkaline conditions. Finally obtained by self-assembly is a solidified material with multiple centers of collagen coated with cyanoacrylate polymers.

The key point of the embolic material lies in that the adhesiveness of the cyanoacrylate enables the embolic body to stick to the vascular wall of the diseased tissue immediately after embolization, ensuring an initial embolic effect; and meanwhile, the collagen not only has a good biocompatibility, but also can promote cell adhesion and fibroplasias, induce the increase of endothelial growth factor, and form a dense connective tissue matrix on the surface of the embolic body, making the embolic body more receptive to the continuous impact of blood flow, and ensuring a long-term and stable embolic effect.

The present invention not only utilizes the dual electron-attracting effect of the cyano group and the carboxyl group in the alkyl cyanoacrylate, enabling the carbon atoms to be rapidly polymerized under the catalysis of anions in the blood, so as to achieve the purpose of embolization, and utilizes the property of alkyl cyanoacrylate being unlikely to polymerize in a 5 % glucose solution, so that the liquid cyanoacrylate monomers can be conveniently injected into the vessels; but also utilizes the hydrophilic groups on the surface of collagen, enabling the collagen to rapidly expand during embolization and self-assemble with the alkyl cyanoacrylate to produce an embolic material for vascular diseases. The multiple-junction polymer formed by self-assembly may dramatically reduce the overall weight of the embolic material and improve the toughness. Furthermore, induction of the increase of endothelial growth factor by collagen provides a long-term and stable embolic effect upon embolization.

In particular, the addition of collagen in the present invention improves the stability of the embolic material at lesions, and provides a basis and carrier for grafting a biological group, which is beneficial for the embolic material to induce the adsorption of biological factors, thereby promoting endothelialization.

The embolic material of the present invention is suitable for vascular diseases, especially aneurysm. This material which can be prepared in a simple method can achieve a stable embolic effect and is capable of reducing the overall weight of the embolized substance with the degradation of degradable components.

The advantages of the present invention are summarized as follows: in one aspect, the alkyl cyanoacrylate, as a typical liquid embolic material, has a good embolic effect; and, in another aspect, the collagen can absorb water and expand rapidly, and can induce the increase of endothelial growth factor upon embolization, which provides an embolic body with a low density and a good flexibility, as well as a permanent embolization with no recurrence. Accordingly, the embolic material of the present invention has a better effect and a higher safety relative to the current embolic agents.

### Embodiments

For a further understanding of the present invention, preferred embodiments of the present invention will be described in conjunction with the following Examples. Such description is to exemplify the features and advantages of the present invention, not limiting the scope of the present invention.

### Example 1

A novel collagen-based vascular embolic material is prepared in accordance with the following procedure:

First, 0.5 mol of collagen is granulated on a fluidized bed (see Jiang Zhao, et al., "Optimization of the Process for Producing Protein Powder with Granulation Technology by Spraying Lecithin on a Fluidized Bed", published in "Food Research and Development", 2007), to obtain collagen particles with a particle size of about 50 mesh, and then added to 3 mol of n-octyl cyanoacrylate monomers, allowing the collagen particles to be uniformly dispersed in the monomers by ultrasonic. Under the atmosphere of argon, 30 g of tantalum nanopowder is added at 37∼50°C and stirred to obtain a homogeneous magnetic fluid of n-octyl cyanoacrylate. The fluid is loaded into a container, sealed and stored after sterilization.

### Example 2

A novel collagen-based vascular embolic material is prepared in accordance with the following procedure:

First, 1 mol of collagen is granulated as described above, and then added to 3 mol of n-octyl cyanoacrylate monomers, allowing the collagen particles to be uniformly dispersed in the monomers by ultrasonic. Under the atmosphere of argon, 20 g of tantalum nanopowder is added at 37∼50°C and stirred to obtain a homogeneous magnetic fluid of n-octyl cyanoacrylate. The fluid is loaded into a container, sealed and stored after sterilization.

### Example 3

A novel collagen-based vascular embolic material is prepared in accordance with the following procedure:

First, 1.5 mol of collagen is granulated as described above, and then added to 3 mol of n-pentyl cyanoacrylate monomers, allowing the collagen particles to be uniformly dispersed in the monomers by ultrasonic. Under the atmosphere of argon, 10 g of tantalum nanopowder is added at 37∼50°C and stirred to obtain a homogeneous magnetic fluid of n-pentyl cyanoacrylate. The fluid is loaded into a container, sealed and stored after sterilization.

The embolic materials obtained in Example 1-3 and onyx glue are used to completely embolize a group of aneurysms in *ex vivo* models through a micro-catheter, and then, the circulation pump is turned on and the temperature is maintained constant at 37°C±1°C, so that the liquid constantly scours the mass of aneurysm after embolization. Two months later, it is found that there is some liquid around the mass embolized by onyx glue, while the embolic body of Examples 1-3 maintain close contact with the wall of aneurysm, with no liquid there-between, indicating that the aneurysm is completely sealed.

In sum, a self-toughening embolic material with biological activity is formed by the self-assembly of collagen and alkyl cyanoacrylate monomers, which not only reduces the occurrence of sticky catheter caused by such embolic agents, but also improves the resultant occupation effect after complete embolization and solidification, as well as the symptoms caused under the inertial compression of aneurysm on the peripheral nerves, by virtue of its toughening effect. More importantly, the combination of collagen and alkyl cyanoacrylate not only results in an instantaneous embolization, but also promotes the formation of a dense connective tissue on the surface of the embolic body by the collagen-induced increase of endothelial growth factor, thus ensuring the safety and reliability of embolization. In addition, the possibility of recurrence is avoided by the close joint between the embolic material and the wall of aneurysm, and the secondary endothelialization at the neck of the aneurysm.

The above description of examples is merely used to help understanding of the central concept of the present invention. It should be noted that several improvements and modifications of the degradable stents of the present invention could be made by one person of ordinary skill in the art without departing from the principle of the present invention. These improvements and modifications also fall within the scope of the present invention set forth in the claims.

## Claims

1. A novel collagen-based liquid embolic material, wherein the alkyl cyanoacrylate and the collagen contained therein self-assemble into a multiple-junction polymer during embolization, to thereby produce an embolic material system.

2. The embolic material according to claim 1, which is further mixed with a vascular developer material.

3. The embolic material according to claim 2, wherein the vascular developer material (contrast agent) is selected from the group consisting of iodide, bismuth-containing compound, metal powder, and any mixture thereof.

4. The embolic material according to claim 3, wherein the iodide is selected from the group consisting of 6-triiodo formic acid, sodium 6-triiodobenzoate, iothalamic acid, metrizoic acid, iodamide, ioxaglic acid, iopanoic acid, iohexol, and iotrolan.

5. The embolic material according to claim 3, wherein the bismuth-containing compound is selected from the group consisting of bismuth trioxide.

6. The embolic material according to claim 3, wherein the metal powder is selected from the group consisting of tantalum powder, gold powder and tungsten powder.

7. A method for preparing the embolic material according to claim 1, comprising:
granulating the collagen, and adding and uniformly dispersing the granulated collagen into alkyl cyanoacrylate monomers, to thereby form a novel liquid embolic material.

8. The preparation method according to claim 7, further comprising adding a vascular developer material.

9. The preparation method according to claim 8, wherein the vascular developer material is a metal powder added at 37-50°C.
